# EUROPEAN PATENT SPECIFICATION

(11) **EP 0 451 429 B1**
(45) Date of publication and mention of the grant of the patent: **24.01.1996**
(21) Application number: 90830594.9
(22) Date of filing: 18.12.1990
(51) Int. Cl.: A61M 1/34

(54) **Blood-purifying equipment**
Einrichtung zur Blutreinigung
Equipement pour la purification du sang

(30) Priority: 09.03.1990 IT 6716890
(43) Date of publication of application: 16.10.1991
(73) Proprietor: BELLCO S.p.A., I-41037 Mirandola (IT)
(72) Inventor: Ghezzi, Paolo, I-12016 Peveragno (Cuneo) (IT); Gervasio, Renzo, I-10132 Torino (IT)
(74) Representative: Bosotti, Luciano

(56) References cited:
- EP-A- 0 111 696
- EP-A- 0 222 709
- DD-A- 206 076
- DE-A- 3 147 377
- FR-A- 2 585 251
- US-A- 3 579 441
- US-A- 4 397 747

## Description

The present invention relates in general to the purification of blood, for example, in patients suffering from renal insufficiency.

In this connection, the solution according to the present invention constitutes a development of the solution described in Italian utility model No. 198528.

In this prior-art solution, which has had considerable clinical success, the blood to be purified is passed through a haemofiltration element and a haemodialysis element which are interconnected in series (in cascade) with respect to the blood flow.

In both elements, the blood to be purified is brought into contact with membranes which have certain degrees of permeability as regards the toxic substances to be eliminated. The toxic substances can pass through the membrane by two distinct physical processes, that is to say, by diffusion or by convection.

In the haemodialysis element, the dominant process is diffusion. In fact, haemodialysis is carried out with membranes constituted, for example, by the material known by the trade name of "Cuprophan" or the like and with a circuit in which the blood to be purified passes along one side of the membranes and a washing solution passes along the other side. The membranes used have high diffusion coefficients for small molecules, such as those of urea, and low diffusion coefficients for medium-large molecules (molecular weights greater than 1000). Their ultrafiltration coefficients are low. The purification achieved by haemodialysis in the patient under treatment is good as regards urea but poor as regards toxic substances with medium-high molecular weights and there is a limited loss of body weight.

The dominant process in the haemofiltration element is convection. In fact, haemofiltration is effected with very porous synthetic membranes and with a blood-flow circuit which creates a pressure gradient through the membrane (currently known as the TMP) and eliminates the ultrafiltrate without the need to use a washing solution. Large quantities of liquid are therefore eliminated (about 20-30 litres during a complete treatment) and both the small and the medium-large molecules are entrained therewith. In order to compensate for the excessive loss of water, it is therefore necessary continuously to infuse solutions of a suitable composition into the patient's blood so as to achieve the desired final loss of body weight.

Combined purifying techniques also exist, such as that known as haemodiafiltration which is comparable to a combination of the haemodialysis and haemofiltration techniques described above and is carried out with membranes similar to those used in haemofiltration. Structurally, this technique is comparable to haemodialysis but, as a result of the greater permeability of the membrane used, the ultrafiltration is more significant (although less than that which occurs in haemofiltration) and it is thus also necessary to infuse a solution of suitable composition into the blood to compensate for the loss.

The solution of the aforesaid IT-U-198528 is based on a combined haemodialysis-haemofiltration technique and has some considerable advantages, such as the fact that the two filter elements can be controlled separately. In fact, the haemodialysis element is connected to normal haemodialysis equipment with a low or zero TMP. The haemofiltration element, however, is associated with a blood-flow circuit which establishes the TMP on the basis of the quantity of ultrafiltrate desired.

The solution of suitable composition which compensates for the excessive loss of body weight in the patient can be infused at a point in the blood-flow circuit intermediate the two filters. Thus, a precise equilibrium of the concentrations of salts in the purified blood and in the washing solution can easily be established in the haemodialysis element which is usually situated downstream. In fact ions (sodium, potassium, etc...) are exchanged easily between the blood and the washing solution through the membranes of the haemodialysis element. In haemofiltration and haemodiafiltration equipment, however, it is much more difficult to achieve a precise control of the salt concentrations in the blood which is returned to the body after purification.

Finally, the overall efficiency of the combined haemodialysis-haemofiltration equipment is very high.

The need to infuse a solution of suitable composition to compensate for the loss of body weight caused by the convection which dominates in haemofiltration, however, creates objective difficulties of an operational nature.

US-A-3579441 discloses equipment for purifying blood by removal of harmful substances contained therein, including at least one filter element through which the flow of blood to be purified is intended to pass to effect a purification step with the formation of a quantity of ultrafiltrate which is removed from the flow and includes the said substances, compensation means for introducing into the blood flow an infusion solution for compensating, at least partially, for the quantity of ultrafiltrate removed, and a regenerator element which is interposed between the filter element and the compensation means so as to act on the ultrafiltrate to remove said substances therefrom, whereby, after the removal of said substances, at least some of the ultrafiltrate can be used to make up the infusion solution. A similar disclosure is present in EP-A-0111696 and US-A-4397747.

As well as obvious problems of cost and storage as far as the structures for treating kidney disease are concerned, the infusion of the solution is not without a risk of pyrogenic effects and the like. Moreover, in any case, there is a certain difficulty in the precise regulation of the electrolytic characteristics of the compensation solution in order to prevent disequilibrium when it is reinfused into the patient's body.

Specifically, the present invention relates to an equipment according to the preamble of Claim 1, which is known, e.g. from IT-U-198528, already referred to in the foregoing. A substantially similar arrangement is hinted at in EP-A-0 222 709.

The object of the present invention is to provide means which solve the aforementioned problems in an ideal manner without foregoing the advantages of the combined haemodialysis-haemofiltration technique described above.

According to the present invention, this object is achieved by virtue of equipment having the further characteristics claimed specifically in Claim 1 which follow. The invention also relates to a corresponding method as claimed in Claim 15.

The invention will now be described with reference to the appended drawings, in which:

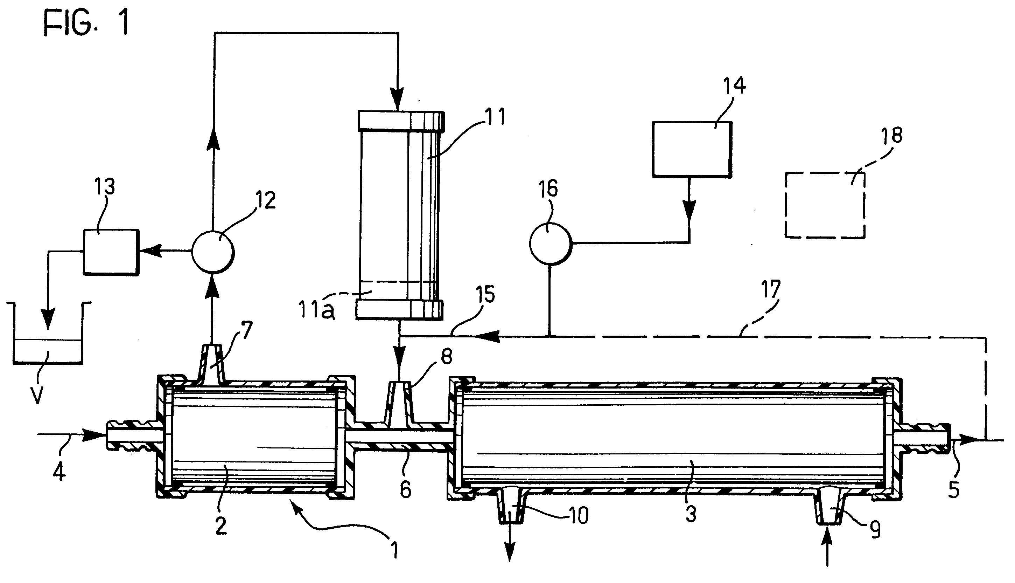

Figure 1 shows the structure of equipment according to the invention in diagrammatic form, and
Figures 2a, 2b, 3a and 3b are graphs showing the efficiency of the purification which can be achieved according to the invention.

In the drawing, equipment for purifying blood by a combined haemodialysis-haemofiltration technique is generally indicated 1.

Essentially, the equipment 1 comprises a haemofiltration element 2 and a haemodialysis element 3 which are arranged in series (in cascade) with respect to the flow of liquid between an inlet connector 4 and an outlet connector 5 for connecting the equipment in a flow-line which draws blood to be purified from the patient's body and subsequently returns the purified blood to the patient's body.

For a more detailed description of these elements reference may usefully be made to the Italian utility model No. 198528 cited above and to the technical solutions claimed therein.

This also applies to the arrangement of a tubular duct 6 which interconnects the elements 2 and 3, actually integrating them in a single device.

In brief, the blood to be purified, which is introduced into the haemofiltration element 2 through the inlet connector 4, passes into the duct 6 after haemofiltration and thence into the haemodialysis element 3 which is situated downstream with respect to the flow of liquid and outputs the purified blood through the outlet connector 5.

The ultrafiltration products (the ultrafiltrate) resulting from the action of the element 2 are output therefrom through an outlet connector 7.

A connector 8, however, enables a reinfusion solution of suitable composition to be introduced into the duct 6 so that it can pass into the haemodialysis element 3 in order partially or wholly to compensate for the losses caused by the ultrafiltration. According to the present invention, this solution is produced by the regeneration of the ultrafiltrate by criteria which will be described further below.

According to a widely-known solution, however, inlet and outlet connectors, indicated 9 and 10 respectively, are provided for the passage of the washing solution for carrying out the haemodialysis process in the element 3.

Essentially, the present invention is based on the realisation that the ultrafiltrate withdrawn from the outlet duct 7 of the element 2 can be regenerated by its being passed through a filter (a regenerator element) 11 so that it can be used as an infusion solution. In the prior art, on the other hand, this solution, which is prepared pharmaceutically, is taken in from outside.

More precisely, the ultrafiltrate output through the connector 7 is supplied to the filter 11 through a pump 12. The pump has a discharge connector 13 for discharging some of the ultrafiltrate to the outside should this be necessary for various reasons (for example, at the start of the purification cycle, etc...). The ultrafiltrate drawn off through the connector 13 is collected in a discharge container V.

A container, indicated 14, is intended to contain electrolytic solutions and/or buffer solutions which may be pumped by a pump 16 through a duct 15 opening into the line leading to the connector 8 so as to be added to the regenerated ultrafiltrate output from the filter 11.

Instead of being sent to the line 15 which ends upstream of the haemodialysis element 3 with respect to the flow of liquid, the solution in the container 14 may be sent through a line 17 which opens downstream of the element 3 before the purified blood is returned to the patient's body.

This also applies to the arrangement of any so-called "buffer free" system 18 for the administration of electrolytes and buffer solutions downstream of the entire haemofiltration-haemodialysis unit. In this case, this takes place through the line 17.

In the solution according to the invention, the ultrafiltrate which is produced by the pressure gradient during the passage of the blood through the element 2 and contains solutes with medium-high molecular weights (including beta-2-microglobulin) is regenerated by its passage through the filter 11. In the embodiment preferred at present, this is constituted essentially by a cartridge of coated or - even better - uncoated activated carbon which can retain the solutes contained in the ultrafiltrate by adsorption.

The filter 11 may, to advantage, be constituted by an (uncoated) carbon haemoperfusion cartridge which has been sold for several years under the trade name of Detoxyl 2 by Sorin Biomedica S.p.a. of Saluggia (Vercelli), Italy.

The ultrafiltrate thus purified can then be used as a reinfusion solution and readministered to the patient.

The very heavy solute molecules thus undergo the following process:
- removal from the blood by haemofiltration,
- purification by adsorption on carbon,
- administration to the patient in purified form.

An "endogenous" replacement liquid containing electrolytes and bicarbonates can be produced and, with obvious advantages, can replace the "exogenous" physiological electrolyte solution (normally prepared pharmaceutically) used in the prior art.

The adsorption is effected on ultrafiltrate which has no red corpuscles, white corpuscles, platelets, fibrinogen, proteins, etc. Thus, the biocompatibility problems which activated carbon has shown towards blood in the past do not arise in the solution according to the invention. These problems have lead some people to propose the use of filter cartridges which have activated carbon coated with various materials (collodion, etc.) but with final results which cannot be considered satisfactory.

Activated (uncoated) carbon shows an excellent ability to adsorb the solutes contained in the ultrafiltrate, particularly as regards beta-2-microglobulin and myoglobin (molecular weights of about 20,000 daltons).

In any case, the risk of active carbon granules being administered to the patient as a result of the reintroduction of the purified ultrafiltrate can be eliminated by a filter element of suitable porosity which may be constituted, for example, by a simple gauze associated with the output of the filter 11. Such a filter is normally provided in the Detoxyl product mentioned above.

In general, the activated carbon of the filter 11 is produced from various natural substances (lignite, anthracite, peat, petroleum, etc.) which have undergone oxidation treatments which give them good adsorption capabilities. The final product is in the form of microgranules, each of which has a multitude of minute channels opening in corresponding pores on its surface. The contact surface developed by virtue of this structure is very large, up to 1000 m² per gram of carbon. The molecules are adsorbed by physico-chemical mechanisms consisting essentially of the trapping of the molecules in the channels. The adsorption spectrum of activated carbon extends to substances with molecular weights of between 100 and 20,000 daltons. The maximum adsorption is of molecules with weights of between 300 and 1500 daltons, identified clinically as "medium molecules", which are present in abnormal quantities in uraemic patients and are incompletely removed by the dialysis membrane (element 3) by diffusion mechanisms.

As already stated, activated carbon in the form described must be considered to have poor biocompatibility and thus to have very poor haemocompatibility. In fact, in contact with the blood, it causes depletion of the erythrocytes, platelets, white corpuscles, fibrinogen and other substances of biological value. Moreover, in circulation, it releases microembolisms which cause rigor, fevers and the accumulation of carbon in the reticuloendothelial systems of the patients.

For these reasons, activated carbon was used sporadically for haemoperfusion until the 60s when Chang proposed and carried out a method of coating the individual granules with a polymer based on albumin and cellulose (collodion). The coatings most generally used are albumin, cellulose and methacrylate. Although, on the one hand, these coating systems provide the activated carbon with good biocompatibility, on the other hand, they restrict its adsorption capabilities since the coating film restricts the passage of solutes with high molecular weights and above all of beta-2-microglobulin whose accumulation in the body a uraemic patient, has great importance in determining the typical pathology of a haemodialysed subject (amyloidosis).

From this point of view, therefore, the present invention goes against the general opinion in the art by proposing the preferential use of uncoated activated carbon to form the filter 11. This is because the filter is not brought into direct contact with the blood but, on the contrary, is only in contact with the haemofiltrate output from the element 2.

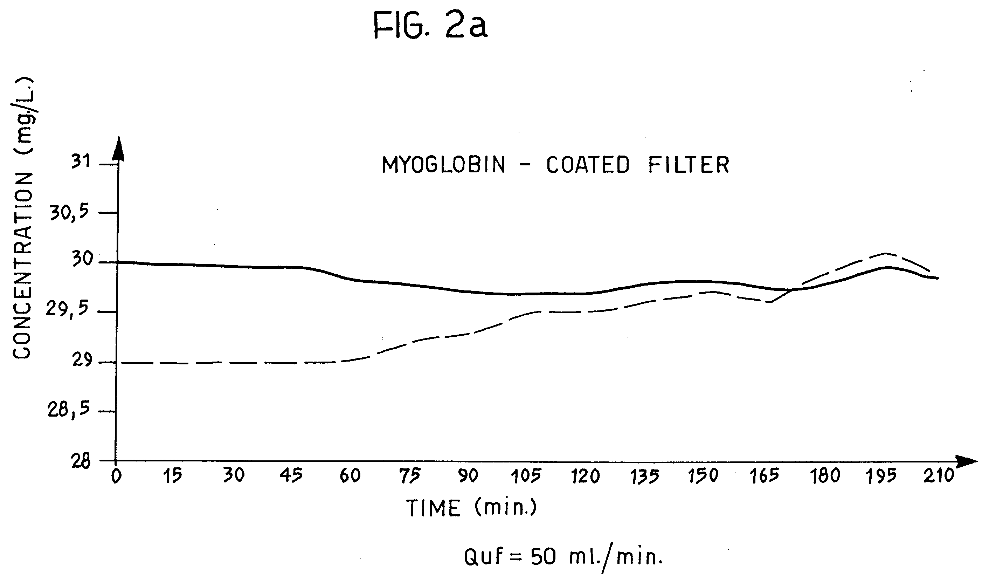

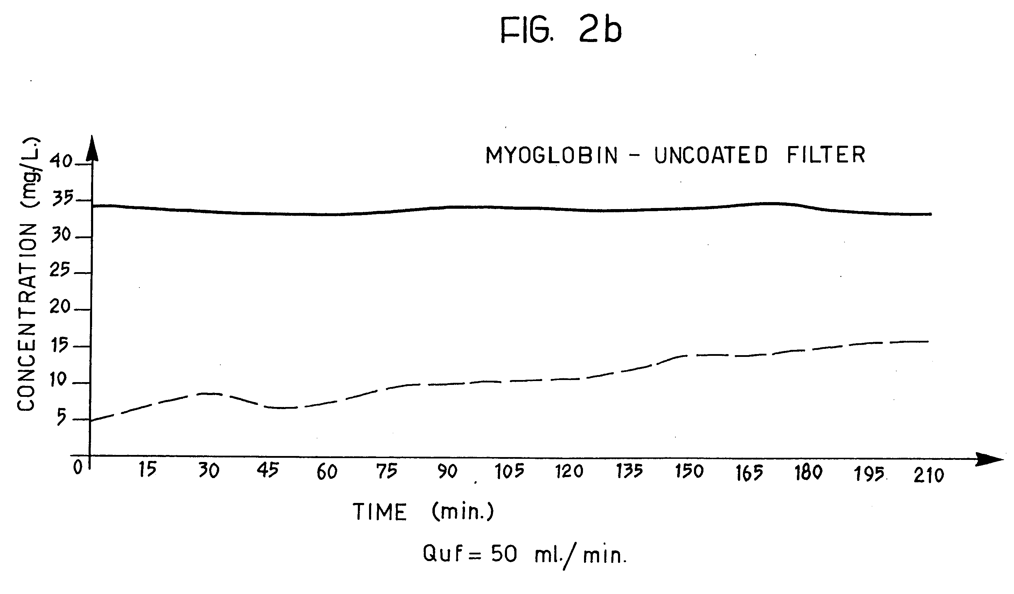

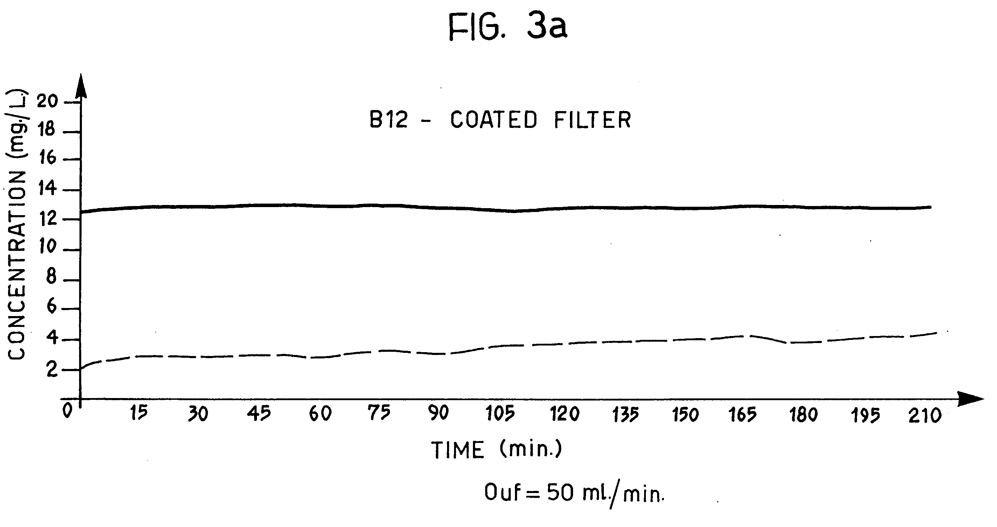

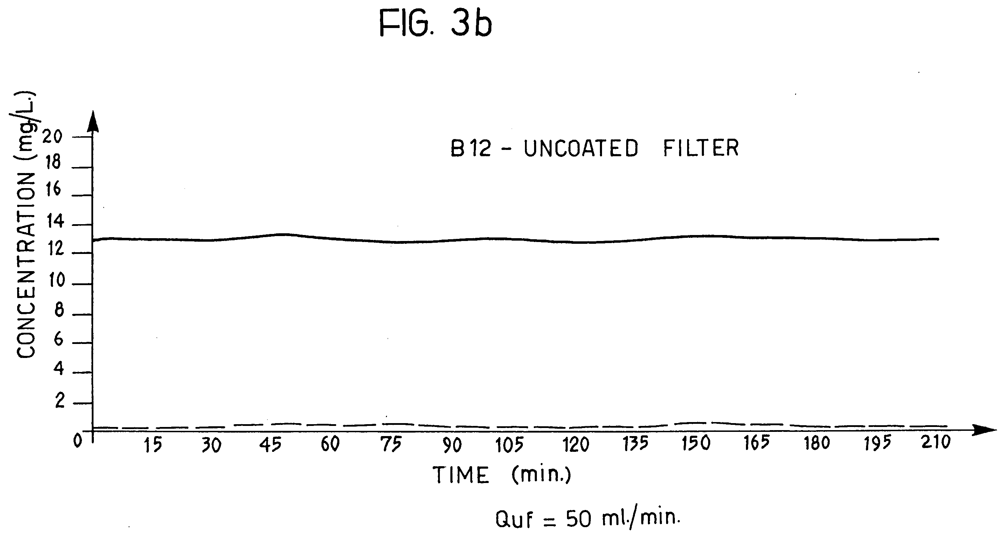

The advantage of the use of this solution is shown specifically by the graphs of Figures 2a, 2b and 3a, 3b which show the percentage adsorption of solutes with high molecular weights (typically myoglobin - Figures 2a and 2b - and vitamin B12 - Figures 3a and 3b) as functions of the treatment time t (in minutes). Each graph shows the variation in the concentration of the solute (in mg/l) at the input (continuous line) and at the output (broken lines) of the filter element 11 respectively. The data relate to a constant flow Quf of 50 ml/minute of solution (in vitro). Figures 2a and 3a show the data relating to the use of a coated carbon filter (300 g), such as the filters sold under the trade name Emoadsorb by Biotech International of Bologna-Italy. Figures 2b and 3b, however, show the results obtained with an uncoated activated carbon filter (Detoxyl 2 - 85 g). The considerable advantages resulting from the use of uncoated activated carbon can readily be appreciated from the graphs.

Naturally, the principle of the invention remaining the same, the details of construction and forms of embodiment may be varied widely with respect to those described and illustrated, without thereby departing from the scope of the present invention. This is particularly true of the criteria for the construction of the filter 11 which may even be formed according to solutions different from those described, for example, with the use of a filter element other than a carbon filter element, for example, a filter operating by reverse osmosis, etc.

## Claims

1. Equipment for purifying blood by removal of harmful substances contained therein, including a first filter element (2) through which the flow of blood to be purified is intended to pass to effect a purification step by haemofiltration with the formation of a quantity of ultrafiltrate which is removed from the flow (7) and includes the said harmful substances as well as a second filter element (3) which is arranged in series with the first filter element (2) with respect to the flow of liquid and through which the flow of blood to be purified is intended to pass to effect a purification step by haemodialysis, and compensation means (8, 17) for introducing into the blood flow an infusion solution for compensating, at least partially, for the quantity of ultrafiltrate removed, characterised in that it includes a regenerator element (11) which is interposed between the first filter element (2) and the compensation means (8, 17) so as to act on the ultrafiltrate to remove said substances therefrom, whereby, after the removal of said substances, at least some of the ultrafiltrate can be used to make up the infusion solution.

2. Equipment according to Claim 1, characterised in that the compensation means (8) are arranged between the first filter element (2) and the second filter element (3).

3. Equipment according to Claim 1, characterised in that the compensation means (17) are arranged downstream of the second filter element (3) with respect to the flow of liquid.

4. Equipment according to Claim 2 or Claim 3, characterised in that the second filter element (3) is downstream of the first filter element (2) with respect to the flow of liquid.

5. Equipment according to any one of Claims 1 to 4, characterised in that discharge means (13) are associated with the first filter element (2) for selectively discharging at least a fraction of the ultrafiltrate from the equipment (V).

6. Equipment according to any one of Claims 1 to 5, characterised in that supply means (14, 16, 18) are associated with the compensation means (8, 17) for supplying a further quantity of infusion solution to be added to the ultrafiltrate output from the regenerator element (11).

7. Equipment according to any one of the preceding claims, characterised in that the regenerator element (11) is a filter.

8. Equipment according to Claim 7, characterised in that the filter works by reverse osmosis.

9. Equipment according to Claim 7, characterised in that the filter (11) works by adsorption.

10. Equipment according to Claim 7 or Claim 9, characterised in that the filter (11) is an activated carbon filter.

11. Equipment according to Claim 7 or Claim 9, characterised in that an auxiliary filter is associated with the filter (11) for retaining any fractions of the material constituting the regenerator element (11) which may be entrained by the ultrafiltrate.

12. Equipment according to Claim 10, characterised in that the filter (11) is a coated carbon filter.

13. Equipment according to Claim 10, characterised in that the filter (11) is an uncoated carbon filter.

14. Equipment according to any one of the preceding claims, characterised in that the regenerator element (11) is intended primarily to remove substances with molecular weights of between about 100 and about 20,000 daltons, preferably between about 300 and about 1500 daltons, from the ultrafiltrate.

15. A method of producing a quantity of an infusion for compensating, at least partially, for a quantity of ultrafiltrate obtained from a quantity of blood by a haemofiltration purification step, in which the ultrafiltrate transports harmful substances to be removed from the blood, wherein in series with the haemofiltration purification step a haemodialysis purification step is effected, said method being characterised in that it includes the step of subjecting the ultrafiltrate to regeneration (11) to remove said substances therefrom so that, after the removal of said substances, at least some of the ultrafiltrate can be used to make up the infusion solution.

16. A method according to Claim 15, characterised in that said haemodialysis purification step (3) is effected on the blood after (8) compensation by the infusion solution.

17. A method according to Claim 15, characterized in that said haemodialysis purification step (3)is effected on the blood before (17) compensation by the infusion solution.

18. A method according to any one of Claims 15 to 17, characterised in that it includes the step of selectively discharging (V) at least a fraction of the ultrafiltrate before the regeneration step (11) is carried out.

19. A method according to any one of Claims 15 to 18, characterised in that it includes the step of adding (14, 16, 18) a further quantity of infusion solution to the ultrafiltrate after the regeneration step (11) has been carried out.

20. A method according to any one of the preceding Claims 15 to 19, characterised in that the regeneration step is carried out by filtration (11).

21. A method according to Claim 20, characterised in that the filtration (11) is carried out by reverse osmosis.

22. A method according to Claim 20, characterised in that the filtration (11) is carried out by adsorption.

23. A method according to Claim 20, characterised in that the filtration (11) takes place through activated carbon.

24. A method according to Claim 23, characterised in that coated carbon is used.

25. A method according to Claim 23, characterised in that uncoated carbon is used.

26. A method according to any one of the preceding Claims 15 to 25, characterised in that it includes the step of removing primarily substances with molecular weights of between about 100 and about 20,000 daltons, preferably between about 300 and about 1500 daltons, from the ultrafiltrate.

## Patentansprüche

1. Gerät zum Reinigen von Blut durch die Entfernung von schädlichen Substanzen, die darin enthalten sind, wobei das Gerät ein erstes Filterelement (2), durch das die zu reinigende Blutströmung geleitet wird, um durch Hämofiltration einen Reinigungsschritt auszuführen, bei dem eine Menge eines Ultrafiltrats erzeugt wird, das von der Strömung (7) entfernt wird und die schädlichen Substanzen aufweist, ein zweites Filterelement (3), das mit dem ersten Filterelement (2) im Hinblick auf die Flüssigkeitsströmung in Serie liegt, und durch das die zu reinigende Blutströmung geleitet wird, um durch Hämodialyse einen Reinigungsschritt auszuführen, sowie eine Kompensationseinrichtung (8, 17) aufweist, um in die Blutströmung eine Infusionslösung einzuleiten, um zumindest teilweise die Menge des Ultrafiltrats zu kompensieren, die entfernt wurde, dadurch gekennzeichnet, daß das Gerät ein Regeneratorelement (11) aufweist, das zwischen dem ersten Filterelement (2) und der Kompensationseinrichtung (8, 17) liegt, um auf das Ultrafiltrat einzuwirken, um die Substanzen davon zu entfernen, wobei nach der Entfernung der Substanzen zumindest ein Teil des Ultrafiltrats für die Herstellung der Infusionslösung verwendet werden kann.

2. Gerät gemäß Anspruch 1, dadurch gekennzeichnet, daß die Kompensationseinrichtung (8) zwischen dem ersten Filterelement (2) und dem zweiten Filterelement (3) angeordnet ist.

3. Gerät gemäß Anspruch 1, dadurch gekennzeichnet, daß die Kompensationseinrichtung (17) im Hinblick auf die Flüssigkeitsströmung stromabwärts des zweiten Filterelements (3) angeordnet ist.

4. Gerät gemäß Anspruch 2 oder 3, dadurch gekennzeichnet, daß das zweite Filterelement (3) im Hinblick auf die Flüssigkeitsströmung stromabwärts des ersten Filterelements (2) liegt.

5. Gerät gemäß irgendeinem der Ansprüche 1 bis 4, dadurch gekennzeichnet, daß dem ersten Filterelement (2) eine Ablaßeinrichtung (13) zugeordnet ist, um wahlweise zumindest einen Teil des Ultrafiltrats vom Gerät (V) abzulassen.

6. Gerät gemäß irgendeinem der Ansprüche 1 bis 5, dadurch gekennzeichnet, daS der Kompensationseinrichtung (8, 17) eine Speiseeinrichtung (14, 16, 18) zugeordnet ist, um eine weitere Menge einer Infusionslösung zuzuführen, die dem Ultrafiltratausgang des Regeneratorelements (11) beigegeben werden soll.

7. Gerät gemäß irgendeinem der bisherigen Ansprüche, dadurch gekennzeichnet, daS das Regeneratorelement (11) ein Filter ist.

8. Gerät gemäß Anspruch 7, dadurch gekennzeichnet, daß der Filter mit Umkehrosmose arbeitet.

9. Gerät gemäß Anspruch 7, dadurch gekennzeichnet, daß der Filter (11) mit Adsorption arbeitet.

10. Gerät gemäß Anspruch 7 oder 9, dadurch gekennzeichnet, daß der Filter (11) ein Aktivkohlefilter ist.

11. Gerät gemäß Anspruch 7 oder 9, dadurch gekennzeichnet, daß dem Filter (11) ein Hilfsfilter zugeordnet ist, um irgendwelche Bruchstücke jenes Materials zurückzuhalten, aus dem das Regeneratorelement (11) besteht und die vom Ultrafiltrat mitgeführt werden können.

12. Gerät gemäß Anspruch 10, dadurch gekennzeichnet, daß der Filter (11) ein beschichteter Kohlefilter ist.

13. Gerät gemäß Anspruch 10, dadurch gekennzeichnet, daß der Filter (11) ein unbeschichteter Kohlefilter ist.

14. Gerät gemäß irgendeinem der bisherigen Ansprüche, dadurch gekennzeichnet, daß das Regeneratorelement (11) in erster Linie dazu dient, um Substanzen mit einem Molekulargewicht zwischen etwa 100 und etwa 20.000 Dalton, vorzugsweise zwischen etwa 300 und 1500 Dalton, vom Ultrafiltrat zu entfernen.

15. Verfahren zur Herstellung einer Menge einer Infusion, um zumindest teilweise eine Menge eines Ultrafiltrats zu kompensieren, das man von einer Blutmenge in einem Hämofiltrations-Reinigungsschritt erhalten hat, wobei das Ultrafiltrat schädliche Substanzen mitführt, die vom Blut entfernt werden sollen, wobei in Serie mit dem Hämofiltrations-Reinigungsschritt ein Hämodialyse-Reinigungsschritt ausgeführt wird, wobei das Verfahren dadurch gekennzeichnet ist, daß es einen Schritt aufweist, um das Ultrafiltrat einer Regeneration (11) zu unterwerfen, um die Substanzen davon zu entfernen, so daß nach dem Entfernen der Substanzen zumindest ein Teil des Ultrafiltrats für die Herstellung der Infusionslösung verwendet werden kann.

16. Verfahren gemäß Anspruch 15, dadurch gekennzeichnet, daß der Hämodialyse-Reinigungsschritt (3) beim Blut nach (8) der Kompensation mit der Infusionslösung ausgeführt wird.

17. Verfahren gemäß Anspruch 15, dadurch gekennzeichnet, daß der Hämodialyse-Reinigungsschritt (3) beim Blut vor (17) der Kompensation mit der Infusionslösung ausgeführt wird.

18. Verfahren gemäß irgendeinem der Ansprüche 15 bis 17, dadurch gekennzeichnet, daß das Verfahren einen Schritt aufweist, um wahlweise zumindest einen Teil des Ultrafiltrats abzulassen (V), bevor der Regenerationsschritt (11) ausgeführt wird.

19. Verfahren gemäß irgendeinem der Ansprüche 15 bis 18, dadurch gekennzeichnet, daß das Verfahren einen Schritt aufweist, um dem Ultrafiltrat eine weitere Menge einer Infusionslösung beizugeben (14, 16, 18), nachdem der Regenerationsschritt (11) ausgeführt wurde.

20. Verfahren gemäß irgendeinem der bisherigen Ansprüche 15 bis 19, dadurch gekennzeichnet, daß der Regenerationsschritt durch Filtration (11) ausgeführt wird.

21. Verfahren gemäß Anspruch 20, dadurch gekennzeichnet, daß die Filtration (11) durch eine Umkehrosmose ausgeführt wird.

22. Verfahren gemäß Anspruch 20, dadurch gekennzeichnet, daß die Filtration (11) durch Adsorption ausgeführt wird.

23. Verfahren gemäß Anspruch 20, dadurch gekennzeichnet, daß die Filtration (11) durch Aktivkohle erfolgt.

24. Verfahren gemäß Anspruch 23, dadurch gekennzeichnet, daß beschichtete Kohle verwendet wird.

25. Verfahren gemäß Anspruch 23, dadurch gekennzeichnet, daß unbeschichtete Kohle verwendet wird.

26. Verfahren gemäß irgendeinem der bisherigen Ansprüche 15 bis 25, dadurch gekennzeichnet, daß das Verfahren einen Schritt aufweist, um vom Ultrafiltrat in erster Linie Substanzen mit einem Molekulargewicht zwischen etwa 100 und etwa 20.000 Dalton, vorzugsweise etwa zwischen 300 und 1500 Dalton, zu beseitigen.

## Revendications

1. Équipement pour purifier du sang par élimination de substances nocives qui y sont contenues, comprenant un premier élément de filtre (2) à travers lequel l'écoulement de sang à purifier est destiné à passer pour effectuer une étape de purification par hémofiltration avec la formation d'une quantité de l'ultrafiltrat qui est retiré de l'écoulement (7) et comprend lesdites substances nocives, ainsi qu'un deuxième élément de filtre (3) qui est placé en série avec le premier élément de filtre (2) du point de vue de l'écoulement de liquide, et à travers lequel l'écoulement de sang à purifier est destiné à passer pour effectuer une étape de purification par hémodialyse, et des moyens de compensation (8, 17) pour introduire dans l'écoulement de sang une solution d'infusion destinée à compenser, au moins partiellement, la quantité d'ultrafiltat retirée, caractérisé en ce qu'il comprend un élément régénérateur (11) qui est intercalé entre le premier élément de filtre (2) et les moyens de compensation (8, 17) de manière à agir sur l'ultrafiltrat pour en retirer lesdites substances, de sorte qu'après le retrait desdites substances, au moins une partie de l'ultrafiltrat peut être utilisée pour constituer la solution d'infusion.

2. Équipement selon la revendication 1, caractérisé en ce que les moyens de compensation (8) sont placés entre le premier élément de filtre (2) et le deuxième élément de filtre (3).

3. Équipement selon la revendication 1, caractérisé en ce que les moyens de compensation (17) sont placés en aval du deuxième élément de filtre (3) du point de vue de l'écoulement de liquide.

4. Équipement selon la revendication 2 ou la revendication 3, caractérisé en ce que le deuxième élément de filtre (3) se trouve en aval du premier élément de filtre (2) du point de vue de l'écoulement de liquide.

5. Équipement selon l'une quelconque des revendications 1 à 4, caractérisé en ce que des moyens d'évacuation (13) sont associés au premier élément de filtre (2) pour évacuer sélectivement au moins une fraction de l'ultrafiltrat hors de l'équipement (V).

6. Équipement selon l'une quelconque des revendications 1 à 5, caractérisé en ce que des moyens d'alimentation (14, 16, 18) sont associés aux moyens de compensation (8, 17) pour amener une autre quantité de solution d'infusion devant être ajoutée à la sortie d'ultrafiltrat depuis l'élément régénérateur (11).

7. Équipement selon l'une quelconque des revendications précédentes, caractérisé en ce que l'élément régénérateur (11) est un filtre.

8. Équipement selon la revendication 7, caractérisé en ce que le filtre fonctionne par osmose inverse.

9. Équipement selon la revendication 7, caractérisé en ce que le filtre (11) fonctionne par adsorption.

10. Équipement selon la revendication 7 ou la revendication 9, caractérisé en ce que le filtre (11) est un filtre au charbon actif.

11. Équipement selon la revendication 7 ou la revendication 9, caractérisé en ce qu'un filtre auxiliaire est associé au filtre (11) pour retenir toute fraction de la matière constituant l'élément régénérateur (11) qui pourrait être entraînée par l'ultrafiltrat.

12. Équipement selon la revendication 10, caractérisé en ce que le filtre (11) est un filtre au charbon traité.

13. Équipement selon la revendication 10, caractérisé en ce que le filtre (11) est un filtre au charbon non-traité.

14. Équipement selon l'une quelconque des revendications précédentes, caractérisé en ce que l'élément régénérateur (11) est destiné principalement à retirer de l'ultrafiltrat des substances ayant un poids moléculaire compris entre environ 100 et environ 20 000 daltons, de préférence entre environ 300 et environ 1500 daltons.

15. Procédé pour produire une quantité d'une infusion pour compenser, au moins partiellement, une quantité d'ultrafiltrat obtenue à partir d'une quantité de sang par une étape de purification par hémofiltration, dans lequel l'ultrafiltrat transporte des substances nocives à retirer du sang, dans lequel une étape de purification par hémodialyse est effectuée en série avec l'étape de purification par hémofiltration, ledit procédé étant caractérisé en ce qu'il comprend l'étape consistant à soumettre l'ultrafiltrat à une régénération (11) pour en retirer lesdites substances, de sorte qu'après le retrait desdites substances, au moins une partie de l'ultrafiltrat peut être utilisée pour constituer la solution d'infusion.

16. Procédé selon la revendication 15, caractérisé en ce que ladite étape de purification par hémodialyse (3) est effectuée sur le sang après la compensation (8) par la solution d'infusion.

17. Procédé selon la revendication 15, caractérisé en ce que ladite étape de purification par hémodialyse (3) est effectuée sur le sang avant (17) la compensation par la solution d'infusion.

18. Procédé selon l'une quelconque des revendications 15 à 17, caractérisé en ce qu'il comprend l'étape consistant à évacuer (V) sélectivement au moins une fraction de l'ultrafiltrat avant que l'étape de régénération (11) soit effectuée.

19. Procédé selon l'une quelconque des revendications 15 à 18, caractérisé en ce qu'il comprend l'étape consistant à ajouter (14, 16, 18) une autre quantité de solution d'infusion à l'ultrafiltrat après que l'étape de régénération (11) a été effectuée.

20. Procédé selon l'une quelconque des revendications précédentes 15 à 19, caractérisé en ce que l'étape de régénération est effectuée par filtration (11).

21. Procédé selon la revendication 20, caractérisé en ce que l'étape de filtration (11) se fait par osmose inverse.

22. Procédé selon la revendication 20, caractérisé en ce que l'étape de filtration (11) se fait par adsorption.

23. Procédé selon la revendication 20, caractérisé en ce que l'étape de filtration (11) se fait par l'intermédiaire de charbon actif.

24. Procédé selon la revendication 23, caractérisé en ce que du charbon traité est utilise.

25. Procédé selon la revendication 23, caractérisé en ce que du charbon non-traité est utilisé.

26. Procédé selon l'une quelconque des revendications 15 à 25 précédentes, caractérisé en ce qu'il comprend l'étape consistant à retirer de l'ultrafiltrat, principalement, des substances ayant un poids moléculaire compris entre environ 100 et environ 20 000 daltons, de préférence entre environ 300 et environ 1500 daltons.
